# FASCICULE DE BREVET EUROPEEN

(11) **EP 2 004 721 B1**
(45) Date de publication et mention de la délivrance du brevet: **08.06.2011**
(21) Numéro de dépôt: 07727728.3
(22) Date de dépôt: 03.04.2007
(51) Int. Cl.: C08G 63/06, C07D 317/36, C08G 63/82, C08G 63/688, C08G 63/685, A61K 47/34

(54) **NOUVEAUX 0-CARBOXY ANHYDRIDES (OCAS) A FONCTION SALIFIABLE ET POLYMERES OBTENUS A PARTIR DE CES OCAS**
NEUE O-CARBOXYANHYDRIDE (OCAS) MIT EINER VERSALZBAREN FUNKTION UND AUS DIESEN OCAS ERHALTENE POLYMERE
NOVEL O-CARBOXY ANHYDRIDES (OCAS) COMPRISING A SALIFIABLE FUNCTION AND POLYMERS OBTAINED FROM THESE OCAS

(30) Priorité: 03.04.2006 FR 0602881
(43) Date de publication de la demande: 24.12.2008
(73) Titulaire: Centre National de la Recherche Scientifique (C.N.R.S.), 75016 Paris (FR); Université Paul Sabatier (Toulouse III), 31400 Toulouse (FR)
(72) Inventeur: BOURISSOU, Didier, F-31830 Plaisance du Touch (FR); THILLAYE DU BOULLAY, Olivier, F-81600 MONTANS (FR); MARTIN-VACA, Bianca, F-31100 Toulouse (FR)
(74) Mandataire: Ahner, Francis
(86) Numéro de dépôt international: PCT/EP2007/053257
(87) Numéro de publication internationale: WO 2007/113304

(56) Documents cités:
- WO-A-2006/037812
- WO-A1-00/02950
- US-A- 3 218 338
- US-B1- 6 267 987
- OUCHI T., FUJINO A.: "Synthesis of poly(alpha-malic acid) and its hydrolysis behavior in vitro" MAKROMOLEKULARE CHEMIE, vol. 190, 1989, pages 1523-1530, XP002407858
- AL-MESFER H., TIGHE B.J.: "polymers for biodegradable medical devices" BIOMATERIALS, vol. 8, 1987, pages 353-359, XP002407859
- SMITH I J ET AL: "Studies in Ring Opening Polymerisation, 6a) Tertiary Base initiated Po" MAKROMOLEKULARE CHEMIE, HUETHIG UND WEPF, BASEL, CH, vol. 182, no. 2, 1981, pages 313-324, XP009049619 ISSN: 0025-116X
- TRIMAILLE T., MÖLLER M., GURNY R.: "Synthesis and ring-Opening polymerization of new monoalkyl-substituted lactides" JOURNAL OF POLYMER SCIENCE : PART A: POLYMER CHEMISTRY, vol. 42, 2004, pages 4379-4391, XP002407860
- DATABASE WPI Week 199039 Derwent Publications Ltd., London, GB; AN 1990-295648 XP002408303 -& JP 02 209918 A (NIPPON SHOJI KK) 21 août 1990 (1990-08-21)

## Description

La présente invention concerne des 1,3-dioxolane-2,4-diones, également appelées O-Carboxy Anhydrides (OCAs), comprenant une fonction salifiable, un procédé de polymérisation contrôlée de ces OCAs et les poly(α-hydroxy acides) obtenus à partir des ces OCAs.

Les poly(α-hydroxy acides) sont des polyesters biodégradables et biocompatibles, particulièrement intéressants pour la chirurgie et la vectorisation de médicaments. En particulier, ils peuvent constituer des biomatériaux, par exemple utiles en tant que matière première pour la fabrication de prothèses, d'implants, ou bien encore pour être mis en oeuvre comme supports permettant la libération de principes actifs. L'application des poly(α-hydroxy acides) à titre de fils de suture résorbables, de substituts cutanés temporaires ou de fibres textiles est également possible.

Dans le domaine des polymères biodégradables destinés au biomédical, que ce soit pour la chirurgie (fils de suture, chirurgie orthopédique etc.) ou la vectorisation de principes actifs, la difficulté est liée au contrôle des propriétés requises pour une application donnée.

Pour la conception de systèmes d'adressage *in vivo* de molécules actives ou plus précisément de transport et de délivrance contrôlés de médicaments au niveau des cibles thérapeutiques visées, il est nécessaire d'élaborer des matériaux dont les dimensions, la structure physique et chimique, permettent tout à la fois le franchissement des différentes barrières physiologiques, la recherche et la reconnaissance de la cible puis son traitement ou sa destruction. La copolymérisation de monomères biocompatibles incluant la stéréocopolymérisation (copolymérisation d'entités énantiomères) et la modification chimique permettent l'adaptation des propriétés d'une substance macromoléculaire. (Vert, M. ; l'Actualité Chimique, nov.-déc. 2003, p. 20-25).

Jusqu'à présent, les poly(α-hydroxy acides) les plus utilisés sont les poly(acide glycolique) (PGA) et poly(L- acide lactique) (PLA), qui sont des polymères non seulement biodégradables, c'est-à-dire clivables sous l'effet de la biochimie du vivant, mais également bioassimilables en raison de la nature même des α-hydroxy acides libérés.

Les PGA et PLA peuvent être préparés par polymérisation des diesters cycliques de l'acide glycolique (glycolide) ou de l'acide L-lactique (L-lactide) ou par polycondensation des hydroxyacides. La voie la plus exploitée semble être la copolymérisation par ouverture de cycle du glycolide et des lactides (L et D) en présence de 2-éthyl hexanoate d'étain (Kowalski, A. ; Libiszowski, J. ; Duda, A. ; Penczek, S. ; Macromolecules, 2000, 33, 1964).

La polymérisation ou copolymérisation par ouverture d'hétérocycles de type diesters d'α-hydroxy acides est limitée en pratique aux acides glycolique et lactiques, ce qui restreint bien sûr les possibilités d'adaptation aux propriétés requises et contraint à de délicates copolymérisations avec d'autres monomères cycliques tels les N-Carboxy Anhydrides dérivés d'amino acides (FR 2 838 964).

Si la copolymérisation et la stéréocopolymérisation des PGA et PLA ouvrent la voie à de nombreux composés macromoléculaires dégradables, ces polymères ne sont généralement pas fonctionnalisés. Or, de nos jours, la diversification des propriétés et la nécessité de répondre à des cahiers des charges de plus en plus exigeants et spécifiques requièrent la synthèse de polymères fonctionnalisés pour couvrir une gamme plus large d'applications thérapeutiques, notamment en pharmacologie.

Les 1,3-dioxolane-2,4-diones, communément désignées sous le terme de O-Carboxy Anhydrides (en abrégé OCAs), sont des hétérocycles à 5 sommets très étudiés dans la littérature en raison de leurs nombreuses applications potentielles. Ils sont par exemple utilisés pour la modification chimique de chaînes latérales d'antibiotiques (Lilly, Eli & Co ; US 3 641 021). Leur préparation par phosgénation des α-hydroxy acides correspondants est connue depuis longtemps, notamment par les travaux de W.H. Davies (*J. Chem. Soc,* **1951,** 1357-1359).

Tous les essais de polymérisation réalisés sur les OCAs, en particulier sur la 5-méthyl-1,3-dioxolane-2,4-dione (dérivée de l'acide lactique), ont conduit de façon aléatoire à des oligomères (masses molaires Mₙ inférieures à 3000 g/mol) comme le montrent bien les travaux de H. R. Kricheldorf et J.M. Jonté (Polym. Bulletin, 1983, 9, 276-281). En dépit de plusieurs catalyseurs basiques testés (pyridine, triéthylamine, t-butylate de potassium, titanate de tétrabutyle), les auteurs précités n'ont synthétisé que des polymères de masse molaire inférieure à 3000 g/mol.

La publication Smith, J ; Tighe, J. ; Makromol. Chem, 1981, 182, 313, décrit la polymérisation de la 5-phényl-1,3-dioxolane-2,4-dione en présence d'une pyridine ou d'une pyridine substituée. Le procédé décrit conduit à la formation d'un polymère ayant une masse molaire moyenne en nombre comprise entre 2 100 et 3 940 g/mol ; l'indice de polydispersité étant compris entre 1,2 et 1,3. Au vu de leurs résultats, les auteurs de cette publication ont remarqué que la masse molaire du polymère obtenu est indépendante de la concentration initiale en pyridine.

En fonction de l'application désirée des poly(α-hydroxyacides), il est souhaitable de pouvoir contrôler la masse molaire du polymère synthétisé.

Ainsi, pour des applications biomédicales comme supports permettant la libération de principes actifs, il est préférable de pouvoir adapter la masse molaire du polymère au type d'application thérapeutique envisagée : par exemple, des masses de 500 à 5000 pour des préparations injectables, des masses de 50 000 à 100 000 pour des patchs. Pour des applications biomédicales comme fils de suture résorbables ou substituts cutanés temporaires, il est préférable que les polymères aient une masse molaire supérieure à 15000 g/mol.

Il est également important de pouvoir fonctionnaliser ces polymères avec des chaînes latérales salifiables. En particulier la présence de chaîne fonctionnalisées dérivées des acides aminés naturels permet de mimer les polyamino acides (ou les protéines) naturels et ainsi d'améliorer l'assimilation et la biocompatibilité de ces composés. Quelques polymères comportant des chaînes carboxylées ou hydroxylées protégées ou non issus de la polymérisation des lactides ont été décrits. Les polymères les plus proches structurellement d'OCAs fonctionnalisés par un groupe salifiable sont les poly-p-malates décrits par Ouchi et Fujino (Makromol. Chem. 1989, 190, 1523). Cependant, le monomère malide dibenzylester n'y est obtenu qu'avec un rendement de 14 % et la conversion lors de son homopolymérisation est au maximum de 15% après pourtant 10 heures de chauffage à 200°C. De plus, la polymérisation des lactides nécessite généralement l'utilisation de catalyseurs à l'étain dont la toxicité est bien connue.

Un OCA à chaîne latérale fonctionnalisée par un acide a déjà été décrit (AI-Mesfer et al, Biomaterials (1987), 8 (5), 353-359). Il s'agit de l'OCA de l'acide tartronique. Toutefois, les essais de préparation d'OCA à partir d'autres acides tels que l'acide malique ont été négatifs et ont conduit exclusivement à l'obtention de l'anhydride malique. De plus la synthèse décrite n'est utilisable que pour l'acide tartronique, pour lequel aucun anhydride correspondant n'est connu, ce qui favorise la synthèse de l'OCA correspondant.

Enfin, il est noté que la polymérisation de l'OCA de l'acide tartronique est très rapide et même trop rapide pour être comparée à celle des OCAs non fonctionnels, ce qui exclut la possibilité d'envisager des copolymérisations. La fonction acide de l'OCA de l'acide tartronique est très proche de la chaîne polymérique et cet OCA diffère donc de ceux découverts par les présents inventeurs. De plus ce document n'utilise pas le procédé de polymérisation selon la présente invention et ne permet donc pas de contrôler la polymérisation des α-hydroxyacides.

Il s'ensuit que l'un des objectifs de l'invention est de fournir de nouveaux OCAs fonctionnalisés par une fonction salifiable et un procédé de synthèse de poly(α-hydroxyacides), à partir de ces OCA, qui permette d'obtenir des produits finis, c'est à dire des polymères comportant cette fonction salifiable, de masse molaire en nombre, Mn, contrôlable. Ces nouveaux OCAs peuvent facilement être copolymérisés avec des OCAs non fonctionnalisés connus.

Les inventeurs ont découvert de nouveaux OCAs fonctionnalisés et un nouveau système catalytique qui permet la préparation de polyesters d'α-hydroxy acides par polymérisation contrôlée d'OCAs fonctionnalisés. L'invention fournit une alternative technique plus générale au procédé classique de fabrication de ces polymères par ouverture de cycle des diesters cycliques à six sommets.

L'invention a pour objet un OCA de formule générale I suivante : 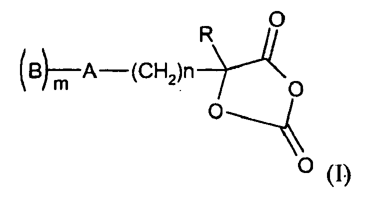 dans laquelle
- n est un nombre entier compris entre 1 et 10, avantageusement entre 1 et 6, de façon avantageuse entre 1 et 4, de façon encore plus avantageuse entre 2 et 4 ;
- A représente un hétéroatome choisi parmi O, N et S ou un radical -COO ou un radical -NH,
- B représente un groupe protecteur de A ;
- m vaut 1 ou 2
- R représente un radical choisi dans le groupe constitué par
   - l'hydrogène,
   - un radical (CH₂)n-A-(B)m dans lequel n, m, A et B sont tels que définis précédemment,
   - les radicaux alkyles en C₁-C₁₂, linéaire ou ramifiés, saturés ou insaturés,
   - les radicaux aralkyles en C₇-C₂₀,
   - les radicaux cycloalkyles, simples ou fusionnés, en C₃-C₁₄,
   - les radicaux hétérocycloalkyles, simple ou fusionnés en C₂-C₁₄,
   - les radicaux aromatiques, simples ou fusionnés, en C₆-C₁₄, et
   - les radicaux hétéroaromatiques, simples ou fusionnés en C₃-C₁₄
   ou leurs sels d'addition, leurs isomères, énantiomères, diastéréoisomères ou leurs mélanges.

Le radical R représente avantageusement un radical choisi dans le groupe constitué par l'hydrogène, les radicaux alkyles, linéaires ou ramifiées, saturés ou insaturés, en C₁-C₆, les radicaux (hétéro)cycloalkyles à 5 ou 6 chaînons et les radicaux (hétéro)aromatiques à 5 ou 6 chaînons. Le radical R représente encore plus avantageusement un atome d'hydrogène.

Au sens de la présente invention, on entend par « groupe protecteur de A », tout groupe protégeant la fonction AH contre les réactions indésirables pendant la polymérisation des OCAs.

Le terme «groupe protecteur » ou « groupe de protection » signifie le groupe qui bloque sélectivement un site réactif (ici le site A) dans un composé multifonctionnel de telle sorte qu'une réaction chimique peut être effectuée sélectivement au niveau d'un autre site réactif non protégé dans la signification classiquement associée à celui-ci en chimie de synthèse.

Dans le cas ou A représente un atome d'oxygène ou un groupe -COO, le groupe protecteur de A est un groupe O-protecteur. Dans ce cas, m vaut 1.

Par le terme "groupe O-protecteur", on entend au sens de la présente invention tout substituant qui protège le groupe hydroxyle ou carboxyle, c'est à dire un atome d'oxygène réactif, contre les réactions indésirables pendant la polymérisation des OCAs tels que les groupes O-protecteur décrits dans Greene, "Protective Groups In Organic synthesis", (John Wiley & Sons, New York (1981)) et Harrison et al. « Compendium of Synthetic Organic Methods", Vols. 1 à 8 (J. Wiley & sons, 1971 à 1996). Les groupes O-protecteur comprennent les éthers ou les esters de méthyle ou d'alkyle substitués ou non, par exemple, méthoxyméthyle, benzyloxyméthyle, 2-méthoxyéthoxyméthyle, 2-(triméthylsilyle) éthoxyméthyle, t-butyle, benzyle et triphénylméthyle, les éthers de benzyle (substitués ou non), les tétrahydropyranyle éthers, les éthers d'allyle, les éthyle éthers substitués, par exemple, 2,2,2-trichloroéthyle, les silyle éthers ou les éthers d'alkylsilyle, par exemple, triméthylsilyle, t-butyldiméthylsilyle et t-butyldiphénylsilyle, les éthers d'hétérocycle; et les esters préparés par réaction du groupe hydroxyle avec un acide carboxylique par exemple, les esters de tert-butyle, de benzyle ou de méthyle, les carbonates en particulier le carbonate de benzyle ou d'halogénoalkyle, l'acétate, le propionate, le benzoate et similaires. Avantageusement il s'agit du groupe benzyle.

Dans le cas ou A représente un groupe NH ou l'hétéroatome N, le groupe protecteur de A est un groupe N-protecteur. Dans ce cas, m vaut 1 ou 2.

Par le terme"groupe N-protecteur", on entend au sens de la présente invention tout substituant qui protège le groupe NH₂ contre les réactions indésirables pendant la polymérisation des OCAs tels que les groupes N-protecteur décrits dans Green, "Protective Groups In Organic synthesis", (John Wiley & Sons, New York (1981)) et Harrison et al. « Compendium of Synthetic Organic Methods", Vols. 1 à 8 (J. Wiley & sons, 1971 à 1996). Les groupes N-protecteur comprennent les carbamates, amides, dérivés N-alkylés, dérivés amino acétale, dérivés N-benzylé, dérivés imine, dérivés énamine et dérivés N-hétéroatome. En particulier, le groupe N-protecteur comprend le formyle, l'acétyle, le benzoyle, le pivaloyle, le phénylsulfonyle, le benzyle (Bn), le t-butyloxycarbonyle (BOC), le benzyloxycarbonyle (Cbz), le p-méthoxybenzyloxycarbonyle, le p-nitrobenzyl-oxycarbonyle, le trichloroéthoxycarbonyl (TROC), l'allyloxycarbonyle (Alloc), le 9-Fluorénylméthyloxycarbonyl (Fmoc), le trifluoro-acétyle, les carbamates de benzyle (substitués ou non) et similaires. Il est avantageux d'utiliser soit du BOC soit du Cbz en tant que groupe N-protecteur en raison de la facilité relative d'élimination, par exemple par des acides modérés dans le cas du BOC, par exemple l'acide trifluoroacétique ou l'acide chlorhydrique dans de l'acétate d'éthyle, ou par une hydrogénation catalytique dans le cas de Cbz. Avantageusement, il s'agit du groupe Cbz.

Dans le cas ou A représente un atome de soufre, le groupe protecteur de A est un groupe S-protecteur. Dans ce cas, m vaut 1.

Par le terme "groupe S-protecteur" on entend au sens de la présente invention tout substituant qui protège le groupe thiol contre les réactions indésirables pendant la polymérisation des OCAs tels que les groupes S-protecteur décrits dans Greene, "Protective Groups In Organic synthesis", (John Wiley & Sons, New York (1981)). Les groupes S-protecteur comprennent les éthers de benzyle (substitués ou non), par exemple le p-méthoxybenzyle ou le p-nitrobenzyle, les éthers de trityle, les thioacétates, les thioacétales et les thioéthers.

Dans la présente invention, on entend désigner par « isomères » des composés qui ont des formules moléculaires identiques mais qui diffèrent par l'agencement de leurs atomes dans l'espace. Les isomères qui différent dans l'agencement de leurs atomes dans l'espace sont désignés par « stéréoisomères ». Les stéréoisomères qui ne sont pas des images dans un miroir l'un de l'autre sont désignés par « diastéréoisomères », et les stéréoisomères qui sont des images dans un miroir non superposables sont désignés par « énantiomères », ou quelquefois isomères optiques. Un atome de carbone lié à quatre substituants non identiques est appelé un « centre chiral ».

« Isomère chiral » signifie un composé avec un centre chiral. Il comporte deux formes énantiomères de chiralité opposée et peut exister soit sous forme d'énantiomère individuel, soit sous forme de mélange d'énantiomères. Un mélange contenant des quantités égales de formes énantiomères individuelles de chiralité opposée est désigné par «mélange racémique».

Dans la présente invention, on entend désigner par « sels d'addition » d'un composé des sels de ce composé formé de la façon suivante :
(1) les sels d'addition d'acide formés avec des acides inorganiques tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires ; ou
(2) les sels formés lorsqu'un proton acide présent dans le composé parent soit est remplacé par un ion métallique, par exemple un ion de métal alcalin, un ion de métal alcalino-terreux ou un ion d'aluminium ; soit se coordonne avec une base organique ou inorganique. Les bases organiques avantageuses comprennent la diéthanolamine, l'éthanolamine, N-méthylglucamine, la triéthanolamine, la trométhamine et similaires. Les bases inorganiques avantageuses comprennent l'hydroxyde d'aluminium, l'hydroxyde de calcium, l'hydroxyde de potassium, le carbonate de sodium et l'hydroxyde de sodium.

Les sels d'addition préférés sont les sels formés à partir d'acide chlorhydrique, d'acide trifluoroacétique, d'acide dibenzoyl-L-tartrique et d'acide phosphorique.

Avantageusement l'OCA selon la présente invention est tel que dans la formule (1), B représente un groupe benzyloxycarbonyle et A représente NH ou B représente un groupe benzyle et A représente O ou un groupe -COO.

De façon avantageuse, l'OCA selon la présente invention est choisi parmis : 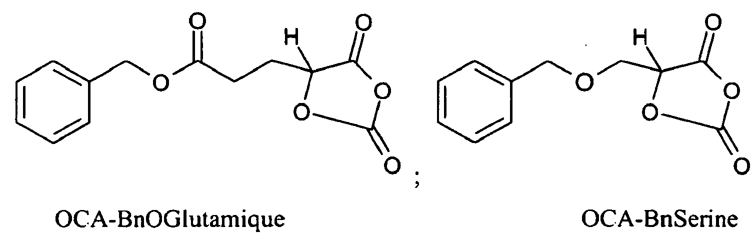 et

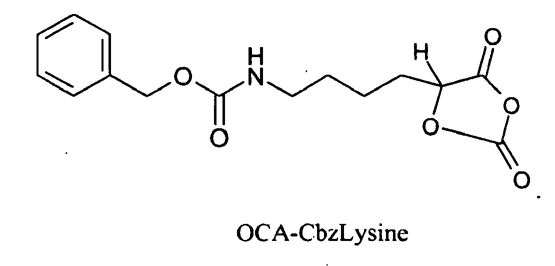

Les OCAs selon la présente invention peuvent être obtenus par phosgénation de l'α-hydroxy acide correspondant, selon des procédés de synthèse connus de l'homme de l'art.

Selon une première variante, les OCAs sont obtenus par phosgénation du sel de lithium de l'α-hydroxy acide de formule (VI) suivante : 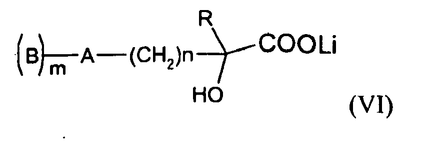 dans laquelle n, A, B, m et R sont tels que définis dans la formule générale (I),
par réaction avec un agent de phosgénation, sans ajout d'une base amine tertiaire, en présence d'un solvant organique non réactif avec l'agent de phosgénation et à une température comprise entre -20°C et +40°C, avantageusement entre -5°C et +30°C, de façon avantageuse entre -20°C et-5°C.

Contrairement aux procédés décrits dans l'art antérieur, la présence d'une base amine tertiaire du type N-méthylmorpholine pour piéger l'acide chlorhydrique est inutile dans le procédé selon cette variante.

Ainsi, dans le cadre du procédé selon cette variante, seuls l'agent de phosgénation, le composé de formule (VI) et le solvant sont nécessaires pour obtenir l'OCA désiré..

Selon une deuxième variante, les OCAs sont obtenus par phosgénation de l'α-hydroxy acide correspondant, ou de l'un de ses sels, en présence d'une base organique amine tertiaire, qui peut éventuellement être supportée (sur du polystyrène par exemple).

La base organique amine tertiaire est de préférence la diisopropyléthylamine.

Les caractéristiques du procédé de synthèse des OCAs, qui suivent, se réfèrent aussi bien à la première variante du procédé qu'à la deuxième variante du procédé. Avantageusement, l'agent de phosgénation est choisi parmi le phosgène, le diphosgène ou le triphosgène, avantageusement le phosgène. De façon avantageuse, les quantités d'agent de phosgénation et de composés de formule (VI) sont équimolaire, ou de façon moins avantageuse l'agent de phosgénation est présent en léger excès par rapport au composé de formule (VI), cet excès ne dépassant pas 10 à 50% du composé de formule (VI).

De façon avantageuse, le solvant est choisi parmi les éthers tels que l'éther diéthylique ou le THF, les hydrocarbures aromatiques tels que le toluène et les esters tels que le formlate de méthyle. De façon encore plus avantageuse, il s'agit du THF ou de l'éther diéthylique.

Avantageusement le temps de réaction est inférieur à 20h, de façon avantageuse compris entre 2h et 6h.

Les composés de formule (VI) quant à eux sont aisément obtenus à partir des amino acides correspondant par des méthodes bien connues de l'homme du métier.

La présente invention a en outre pour objet l'utilisation d'un OCA de formule générale (I) selon la présente invention pour la préparation d'un poly(α-hydroxyacides).

La présente invention a de plus pour objet un procédé de préparation de poly(α-hydroxyacides) comprenant les étapes successives suivantes :
i) polymérisation de manière contrôlée de monomères d'OCAs de formule (I) selon la présente invention dans un solvant organique, avantageusement anhydre, à une température comprise entre -20 et 200°C, avantageusement entre 0 et 100°C, encore plus avantageusement entre 20 et 50°C, en présence d'un système catalytique comprenant une base, ladite base étant un hétérocycle aromatique à 5 ou 6 chaînons comprenant au moins un atome d'azote endocyclique, sous réserve que lorsque la base est utilisée seule dans le système catalytique, elle ne représente pas la pyridine, la 2-méthylpyridine, la 2,6-diméthylpyridine ou la 2-méthoxypyridine; puis
ii) le cas échéant purification du polymère obtenu à l'étape i),
iii) récupération du polymère obtenu à l'étape i) ou ii),
iv) éventuellement protection du groupe OH terminal du polymère obtenu à l'étape iii) avec un groupe O-protecteur différent de B ;
v) éventuellement déprotection du ou des groupe(s) A du polymère obtenu à l'étape iv) afin d'obtenir le ou les groupe(s) AH ;
vi) éventuellement déprotection du groupe OH terminal du polymère obtenu à l'étape v).

Le terme « déprotection » ou « déprotégé(s) » signifie le procédé par lequel un groupe protecteur est éliminé après qu'une réaction sélective sur un groupe non protégé est achevée. Certains groupes protecteurs peuvent être préférés par rapport à d'autres en raison de leur commodité ou de leur facilité relative d'élimination. Les réactifs déprotecteurs pour des groupes hydroxyle ou carboxyle protégés comprennent les carbonates de potassium ou de sodium, l'hydroxyde de lithium dans des solutions alcooliques, le zinc dans du méthanol, l'acide acétique, l'acide trifluoroacetique, les catalyseurs au palladium, l'hydrogène en présence de catalyseurs au palladium, l'acide chlorhydrique ou sulfurique, la soude et les bases organiques (par exemple la pipéridine pour enlever le groupe Fmoc), ou le tribromure de bore, et similaires.

Selon une caractéristique essentielle de l'invention, la réaction de polymérisation de l'étape i) est réalisée de manière contrôlée, c'est-à-dire que la masse molaire, en nombre ou en poids, du polymère obtenu à la fin de la réaction de synthèse peut être déterminée à l'avance en réglant la quantité molaire introduite de système catalytique par rapport à la quantité molaire initiale de monomère, c'est-à-dire en réglant le rapport molaire initial monomère/(système catalytique). En effet, les inventeurs ont découvert que la masse molaire, en nombre ou en poids, du polymère obtenu est une fonction quasi-linéaire du rapport molaire initial monomère/(système catalytique), le coefficient directeur dépendant du solvant dans lequel la réaction se déroule (effet solvant). On peut ainsi synthétiser facilement des polymères de masse molaire en nombre supérieure à 3 000 g/mol en introduisant la quantité molaire nécessaire de système catalytique, par rapport à la quantité molaire initiale de monomère.

Plus on désire obtenir un polymère de masse molaire élevée, plus le rapport molaire initial monomère/(système catalytique) doit être élevé, c'est-à-dire que plus la quantité molaire introduite de système catalytique doit être faible par rapport à la quantité molaire initiale de monomère.

Selon une variante avantageuse de l'invention, le procédé de polymérisation est caractérisé en ce que le rapport molaire monomère/(système catalytique) est supérieur à 10, avantageusement compris entre 20 et 1000, encore plus avantageusement compris entre 50 et 1000, encore plus avantageusement compris entre 120 et 1000, encore plus avantageusement compris entre 200 et 1000.

La base présente dans le système catalytique selon l'invention est avantageusement un hétérocycle aromatique à 5 ou 6 chaînons comprenant au moins un atome d'azote endocyclique conjugué avec un autre atome d'azote endo ou exocyclique.

Selon une variante de l'invention, ladite base est une amino-pyridine de formule (II) 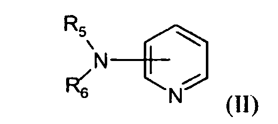 dans laquelle R₅ et R₆ représentent indépendamment l'un de l'autre un radical allyle en C₁-C₆, linéaire ou ramifié, saturé ou insaturé, ou R₅ et R₆ forment ensemble un hétérocycloalkyle à 5 ou 6 chaînons, le groupe -NR₅R₆ étant en position 2 ou 4.

Le noyau des pyridines peut également être substitué par un ou plusieurs radicaux alkyles en G₁-C₆.

En particulier, ladite base est la 4-diméthylamino pyridine (DMAP).

Selon une autre variante de l'invention, ladite base est avantageusement un imidazole de formule (III) 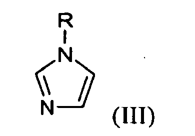 dans laquelle R représente un radical alkyle en C₁-C₆ linéaire ou ramifié, saturé ou insaturé.

Le noyau imidazole peut également être substitué par un ou plusieurs radicaux alkyles en C₁-C₆.

En particulier, ladite base est le N-méthyl-imidazole.

Selon une variante avantageuse de l'invention, le système catalytique selon l'invention comprend uniquement une base. Dans ce cas, le radical Z, dans la formule semi développée du polymère, représente l'atome d'hydrogène et X représente l'atome d'oxygène.

Le rapport molaire initial monomère/base est avantageusement supérieur à 10, encore plus avantageusement compris entre 20 et 1000, encore plus avantageusement compris entre 50 et 1000, encore plus avantageusement compris entre 120 et 1000, encore plus avantageusement compris entre 200 et 1000.

Selon une autre variante avantageuse de l'invention, le système catalytique comprend en outre un réactif protique.

Au sens de la présente invention, on entend par l'expression « réactif protique » tout réactif qui comporte des atomes d'hydrogène susceptibles d'être libérés sous forme de protons.

Le réactif protique permet, grâce à la libération d'au moins un proton, d'amorcer la réaction de polymérisation. Une fois la réaction de polymérisation amorcée, elle est autoentretenue grâce à la fonction alcool de l'oligomère ainsi formé.

La réaction de polymérisation jusqu'à l'étape i) peut alors s'écrire :

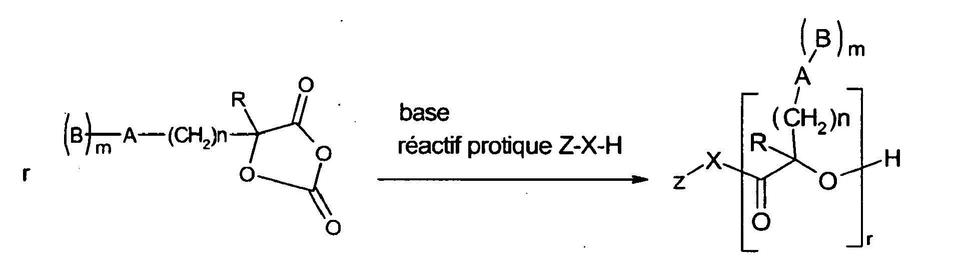

Z représentant le reste du réactif protique et X représentant un hétéroatome choisi dans le groupe constitué par N, O et S.

Dans le cadre de la présente invention, le réactif protique est avantageusement choisi dans le groupe constitué par l'eau, les alcools, les amines primaires et secondaires, les thiols et les polymères à fonctionnalité alcool, amino ou thiol.

Dans le cadre de la présente invention, tout alcool, primaire, secondaire ou tertiaire, peut être utilisé comme réactif protique. Par exemple, on peut utiliser l'éthanol, le pentanol, la Boc-éthanolamine, l'alcool néopentylique ou des alcools de structure plus complexe. De même, toute amine primaire ou secondaire peut être utilisée dans le cadre de la présente invention. Par exemple, on peut citer la benzylamine, l'héxylamine, l'oleylamine, l'éthanolamine, les aminoacides basiques tels que la lysine ou les peptides C-protégés ou des amines de structure plus complexe. Tout thiol peut également être utilisé comme réactif protique. Par exemple, on peut utiliser le benzylthiol, les dérivés de la cystéine.

Dans le cadre de la présente invention, l'expression « polymères à fonctionnalité alcool, amino ou thiol » désigne tous les polymères porteurs de substituants comportant au moins une fonction réactive choisie dans le groupe constitué par les fonctions -OH, -NH₂ et -SH.

Le réactif protique est avantageusement choisi dans le groupe constitué par l'eau, les alcools aliphatiques en C₁-C₁₂, les poly(acide glycolique), les poly(acide lactique) et leurs copolymères.

Selon une variante avantageuse de l'invention, le réactif protique est choisi dans le groupe constitué par les poly(acide glycolique), les poly(acide lactique) et leurs copolymères. On obtient alors des copolymères blocs (lactide et/ou glycolide)-OCA.

Le réactif protique, présent dans le système catalytique, permet un meilleur contrôle de l'indice de polydispersité (Ip) (dispersion en masse autour de la masse moyenne), qui est plus proche de 1, et il favorise également le contrôle de la masse du polymère.

Lors de l'étape i), le solvant est avantageusement choisi dans le groupe constitué par les solvants aliphatiques chlorés, les éthers, les éthers cycliques, les aromatiques, les amides ou les lactames (par exemple la N-methylpyrrolidone)..

Le polymère obtenu suite à l'étape i) peut le cas échéant être purifié lors de l'étape ii) puis le polymère est récupéré lors de l'étape iii).

Ces opérations de purification et de récupération du polymère sont réalisées de manière classique, par exemple par élimination du solvant, par évaporation sous pression réduite ou par concentration ou non du milieu réactionnel, suivie d'une précipitation par addition d'un non solvant tel qu'un alcane en C₁-C₆ ou par lavage biphasique.

Les étapes iv) à vi) permettent le clivage des groupes protecteurs des fonctions salifiables (A-(B)m) afin d'obtenir un polymère dont les fonctions salifiables ne sont plus protégées (AH).

En particulier, afin de déprotéger ces fonctions salifiables (étape v), il convient avantageusement au préalable de protéger la fonction hydroxyle terminale du polymère obtenu à l'étape iii) (étape iv). Cette protection doit être effectuée avec un groupement O-protecteur différent de B, avantageusement avec un acétyle. Cette étape de protection est donc réalisée par des procédés bien connus de l'homme du métier.

Selon le groupe protecteur B utilisé, l'étape v) est réalisée par des méthodes bien connues de l'homme du métier. Ainsi, dans le cas où B représente le groupe Bn, la déprotection peut avoir lieu par hydrogénation catalytique du polymère obtenu à l'étape iv), avantageusement en présence du catalyseur Pd/C à 10%.

L'étape facultative vi) permet de récupérer un polymère dont la fonction terminale OH n'est plus protégée. Elle est réalisée par des procédés bien connus de l'homme du métier.

La présente invention concerne donc les polymères Poly(α-hydroxyacide) obtenus par le procédé selon la présente invention.

On obtient ainsi des polymères et matériaux possédant les caractéristiques dimensionnelles, mécaniques, chimiques, biochimiques et biologiques requises pour toutes les applications envisagées des polyesters dérivés des α-hydroxy acides :
- implants, dentisterie, endoprothèses, chirurgie orthopédique,
- systèmes d'adressage *in vivo* de molécules actives, c'est-à-dire de transport et de délivrance contrôlés de principes actifs au niveau des cibles biologiques choisies, que ce soit dans le domaine médical ou cosmétique.

L'invention permet de résoudre de manière efficace les problèmes posés par la mise en oeuvre obligée de la méthode de l'art intérieur
- possibilité de polymériser et copolymériser un très grand nombre d'α-hydroxy acides comportant des groupes fonctionnels convenablement protégés.
- augmentation et harmonisation des vitesses de polymérisation ce qui est recherché, non seulement pour des questions de coût, mais aussi pour préparer des copolymères « statistiques » ou « blocs ».
- accès à des polymères « sur mesure », c'est-à-dire possédant les caractéristiques physiques et les structures chimiques désirées et dont les groupes pendants sont salifiables.

Les polymères obtenus par le procédé selon l'invention ont avantageusement un indice de polydispersité compris entre 1 et 2, plus avantageusement compris entre 1 et 1,5, et encore plus avantageusement compris entre 1 et 1,4. Les polymères obtenus par le procédé selon l'invention ont donc une courbe de distribution des masses molaires resserrée, les masses molaires sont donc peu dispersées. Plus l'indice de polydispersité a une valeur proche de 1, plus le nombre de macromolécules formées qui ont la même masse molaire est important.

Dans le cas d'utilisation de polymères obtenus par le procédé selon l'invention dans des systèmes d'adressage *in vivo* de molécules actives, il est particulièrement avantageux que ces polymères aient un indice de polydispersité proche de 1.

Un indice de polydispersité voisin de 1 confirme le caractère « vivant » de la polymérisation, soit l'absence de réactions secondaires et la bonne définition des polymères comportant des terminaisons actives. L'étroitesse de la distribution moléculaire peut en outre permettre d'ajuster plus précisément la composition en masse de la préparation médicamenteuse en fonction de la cible désirée et des mécanismes biologiques impliqués. Par exemple, dans les préparations injectables, il est préférable que les molécules soient de même taille, en particulier pour faciliter le passage dans les capillaires. Enfin, d'un point de vu réglementaire, il est plus facile de faire homologuer par les agences de médicaments des principes actifs monomoléculaires.

Selon une variante de l'invention, les polymères obtenus par le procédé selon l'invention ont avantageusement une masse molaire moyenne en nombre supérieure à 1 000 g/mol, encore plus avantageusement supérieure à 2 000 g/mol.

La présente invention concerne également de nouveaux polymères, susceptibles d'être obtenus par le procédé selon l'invention, de formule (IV) 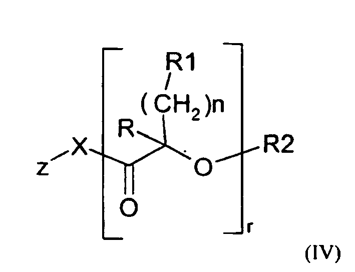 dans laquelle
- n et R ont la même définition que pour l'OCA de formule (I) ;
- R1 représente le groupe -A-(B)m ou le groupe -A-H, dans lesquels A, B et m ont la même définition que pour l'OCA de formule (I);
- R2 représente un groupe O-protecteur différent de B ou un atome d'hydrogène ;
- X représente un hétéroatome, choisi dans le groupe constitué par O, N et S, avantageusement O, ou un polymère choisi dans le groupe constitué par les poly(acide glycolique), les poly(acide lactique) et leurs copolymères, ledit polymère étant terminé par un radical -X' dans lequel X' représente un hétéroatome, choisi dans le groupe constitué par O, N et S, avantageusement O;
- Z représente un radical alkyle en C₁-C₁₂, linéaire ou ramifié, saturé ou insaturé
- r est supérieur ou égal à 1.
r est avantageusement compris entre 1 et 500, encore plus avantageusement compris entre 1 et 400, encore plus avantageusement compris entre 11 et 350.

Selon une variante avantageuse de l'invention, dans la formule (IV), X représente l'héléroatome O et Z représente un radical alkyle en C₁-C₆.

Selon une autre variante avantageuse de l'invention, dans la formule (IV), -X-Z représente un radical de formule (V) 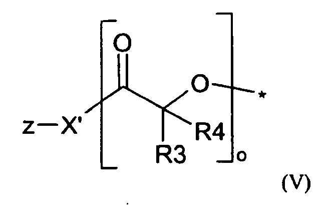 dans laquelle o est supérieur ou égal à 1, R3 et R4 représentent H ou un radical alkyle en C₁-C₆, avantageusement CH₃, cycloalkyle en C₃-C₆ ou aryl(alkyle en C₁-C₆), X' représente un hétéroatome choisi dans le groupe constitué par O, N et S, avantageusement O, et Z représente un radical alkyle en C₁-C₁₂, linéaire ou ramifié, saturé ou insaturé. Avantageusement, X' représente l'hétéroatome O et Z représente un radical alkyle en C₁-C₆.

Les polymères de formule (IV) ont avantageusement un indice de polydispersité compris entre 1 et 2, plus avantageusement compris entre 1 et 1,5 et encore plus avantageusement compris entre 1 et 1,4.

Selon une variante de l'invention, les polymères de formule (IV) ont avantageusement une masse molaire moyenne en nombre supérieure à 1 000 g/mol, encore plus avantageusement supérieure à 2 000 g/mol.

La présente invention concerne enfin l'utilisation des polymères susceptibles d'être obtenus par le procédé selon l'invention pour la vectorisation de principes actifs et pour la fabrication de biomatériaux.

Les exemples suivants permettent d'illustrer l'invention et ils ne sont pas limitatifs. Sauf indication contraire, dans les exemples qui suivent :
o le pentanol et le THF utilisés ont été distillés sur sodium. Le DCM a été distillé sur P₂O₅. La DMAP a été recristallisée dans le toluène ; Le Lac-OCA est recristallisé dans l'éther éthylique puis sublimé. Le BnGlu-OCA est recristallisé dans un mélange iPr₂O/Et₂O 2/1.
o les masses moyennes en nombre (Mn) et en poids (Mw) ont été déterminées par GPC dans le THF (pompe Waters 600 ; détecteur par indice de réfraction Waters 2410 ; colonnes Waters Styragel HR1). Des étalons de polystyrène de faible indice de polydispersité ont été utilisés pour établir la courbe d'étalonnage ;
o les spectres de RMN ¹H sont enregistrés dans le CDCl₃ (spectromètre Brucker à 300 MHz).

### Exemple 1 : Synthèse du BnOGlu-OCA

### Synthèse de l'acide γ-benzyl-2-hydroxyglutarique : (Deechongkit, S. et al., Org. Lett 2004, 6, 497-500; Civitello, E. R. et al., J. Org. Chem. 1994, 59, 3775-3782)

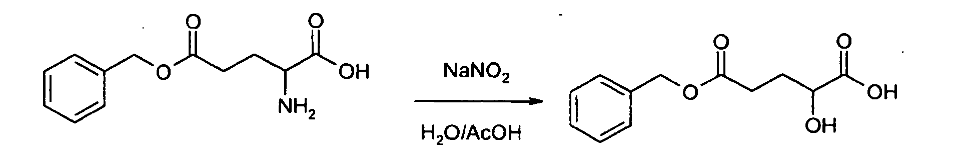

Ajouter en 30 min à 0°C 10 mL de solution de NaNO₂ 2M (20,0 mmol) à une suspension de L-BnOGlu (2,37 g; 10,0 mmol) dans 100 mL d'un mélange H₂O/AcOH 8/2. Agiter 4h à température ambiante, le milieu réactionnel est alors homogène. Ajouter 100 mL d'eau et extraire à l'AcOEt (3 x 50 mL). Laver à la saumure puis sécher sur sulfate de sodium. Evaporer le solvant pour obtenir 2,70 g d'huile visqueuse. Le brut est purifié par chromatographie flash (100 g de silice). Eluant : DCM 95/ MeOH 4,5 / AcOH 0,5. Récupérer une huile légèrement colorée (1,38 g, 58%).
RMN ¹H (CDCl₃ - 300 MHz): 7,35 (m, 5H) ; 5,13 (s, 2H) ; 4,31 (dd, 1H, J_{HH} = 7,6 et 3,9 Hz) ; 2,6 (m, 2H); 2,0-2,3 (m, 2H).
RMN ¹³C (CDCl₃-75 MHz) : 171,2 ; 166,6 ; 147,9 ; 135,2 ; 128,7-128,2 ; 78,0 ; 67,1 ; 28,4 ; 26,0.

### Synthèse de l'OCA-glutamique :

### Protocole N°1 :

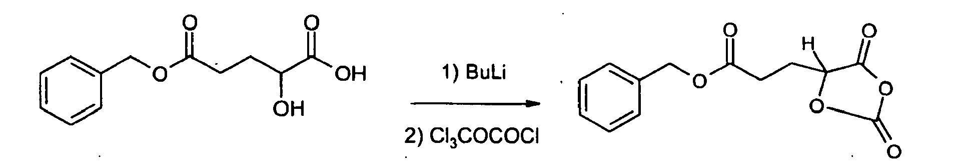

A une solution d'hydroxyacide (4,20 mmol ; 1,0 g) dans l'éther diéthylique anhydre ajouter en 15 minutes et à -30°C une solution de BuLi (4,20 mmol). Le milieu réactionnel devient rapidement hétérogène. Agiter 1 heure à -20°C. Ajouter à -20°C le diphosgène (4,20 mmol ; 0,50 mL) à la suspension de sel de lithium. Agiter le milieu réactionnel 2,5 heures à - 20°C puis contrôler par RMN ¹H. Les sels de lithium insolubles sont éliminés par filtration sous atmosphère inerte et rincés par 10 mL d'éther diéthylique anhydre. Les solvants sont éliminés sous pression réduite et l'huile obtenue est rincée au pentane (2 x 10 mL). Le résidu huileux est trituré dans de l'éther diisopropylique. Le produit attendu cristallise dans le milieu sous forme de cristaux blancs (0,41 g, 37%).

### Protocole N°2 :

Ajouter à 0°C la diisopropyléthylamine (6,30 mmol ; 1,10 mL) à une solution d'hydroxyacide (6,30 mmol ; 1,50 g) dans du THF anhydre. Agiter le milieu réactionnel 30 min à température ambiante. Ajouter à 0°C le diphosgène (6,30 mmol ; 0,76 mL) à la solution précédente. Agiter le milieu réactionnel lh à 0°C puis 3h à température ambiante. Evaporer le THF. Laver l'huile obtenue par du pentane (2 x 15 mL). Reprendre le résidu huileux à l'éther diéthylique et éliminer les insolubles. Evaporer l'éther diéthylique sous pression réduite pour obtenir une huile qui cristallise (0,99 g, 59%).

### Protocole N°3 :

Ajouter à 0°C le diphosgène (3,0 mmol, 0,36 mL) à une suspension de sel de dicyclohexylamine de l'hydroxyacide (3,0 mmol, 1,26 g) et de diisopropyléthylamine greffée sur polystyrène (1,0 g, 3,0 mmol) dans 20 mL d'éther diéthylique anhydre. Agiter le milieu réactionnel 4h à température ambiante. Filtrer la résine, rincer par 15 mL d'éther diéthylique anhydre. Rassembler les phases éthérées puis évaporer le solvant. Laver l'huile obtenue au pentane (2x10 mL). Sécher le produit sous pression réduite pour obtenir une huile qui cristallise au congélateur (0,53 g, 67%).
RMN ¹H (CDCl₃ - 200 MHz): 2,44-2,22 (m, 2H) ; 2,63 (t, 2H, J_{HH} = 6,7 Hz) ; 5,14 (s, 2H) ; 5,21 (dd, 1H, J_{HH} = 7,8 et 5,4 Hz) ; 7,36 (m, 5H).
RMN ¹³C (CDCl₃ - 75 MHz) : 26,0 ; 28,3 ; 67,1 ; 78,0 ; 128,2-128,7 ; 135,2 ; 147,9 ; 166,6 ; 171,2.
Point de fusion : 59-60°C
IR(KBr): 1891; 1805; 1740
MS (IE) : 264 ; 236 ; 210 ; 174 ; 108 ; 91 ; 65. (M = 264,23)

### Exemple 2 : Polymérisation du BnGlu-OCA obtenu à l'exemple 1

### Homopolymérisation :

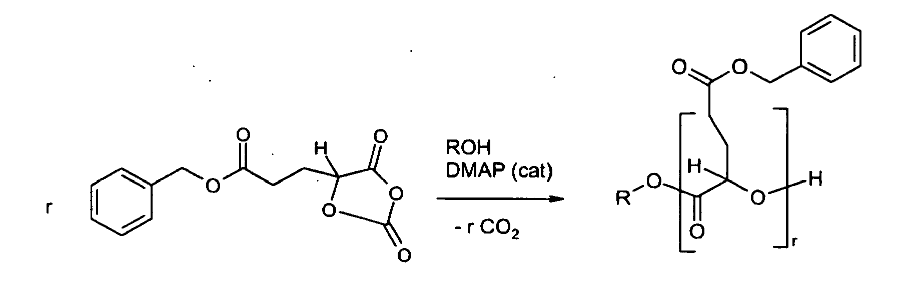

### - poly(BnGlu)₂₀

Dans un tube de schlenk préalablement séché sous vide, mettre en solution le BnGlu-OCA obtenu à l'exemple 1 (1,58 mmol, 370 mg) dans 5 mL de DCM. Ajouter sous agitation et à température ambiante le n-pentanol (0,079 mmol, 9 µL) puis la DMAP (0,079 mmol, 10 mg). Après 5 min (fin du dégagement de CO₂) la consommation totale du monomère est confirmée par RMN ¹H. Ajouter 5 mL de DCM et laver le milieu réactionnel par 2 fois 10 mL d'HCl 2N et 10 mL de saumure. Sécher sur sulfate de sodium anhydre et évaporer à siccité pour obtenir une huile visqueuse (120 mg, 75%).
RMN ¹H : 7,30 (m, **Ar**) ; 5,10 (m, **CH₂**Ar + **CH**OCO) ; 4,29 (dd, J_{HH}=4,2Hz, J_{HH}=7,8Hz, **CH**OH); 4,04 (t, J_{HH}=6,8Hz, O**CH₂**CH₂) ; 2,0-2,6 (m, **CH₂CH₂**CO₂Bn) ; 1,50 (m, OCH₂**CH₂**CH₂) ; 1,23 (m, **CH₂CH₂**CH₃) ; 0,83 (t, J_{HH}=6,8Hz, CH₂CH₂**CH₃**). Incorporation d'un motif n-pentanol pour 20 motifs glutamiques.
GPC : Mn=2700 ; Mw=3380 ; IP=1,25

### - poly(BnGlu)₅₀

Dans un tube de schlenk préalablement séché sous vide, mettre en solution le BnGlu-OCA obtenu à l'exemple 1 (1,05 mmol, 277 mg) dans 4 mL de DCM. Ajouter sous agitation et à température ambiante une solution de 0,05M d'alcool néopentylique dans le DCM (21 µmol, 140 µL) puis une solution de 0,066M de DMAP dans le DCM (21 µmol, 140 µL). Après 15 min (fin du dégagement de CO₂) la consommation totale du monomère est confirmée par RMN ¹H. Ajouter 5 mL de DCM et laver le milieu réactionnel par 2 fois 10 mL d'HCl 2N et 10 mL de saumure. Sécher sur sulfate de sodium anhydre et évaporer à siccité pour obtenir une huile visqueuse (210 mg, 91%).
RMN ¹H : 7,30 (m, **Ar**) ; 5,10 (m, **CH₂**Ar + **CH**OCO) ; 3,82 (s, O**CH₂**tBu) ; 2,0-2,6 (m, **CH₂CH₂**CO₂Bn) ; 0,90 (s, OCH₂**tBu**). Incorporation d'un motif alcool pour 50 motifs glutamiques.
GPC : Mn=6290 ; Mw=7440 ; IP=1,18

### - poly(Glu)₁₀OAc

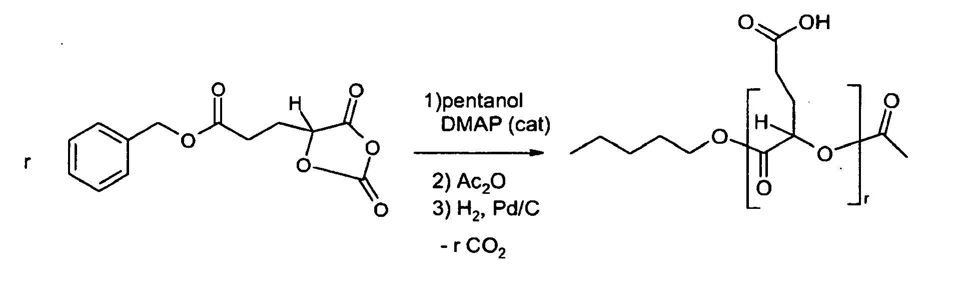

### 1) Polymérisation

Dans un tube de schlenk préalablement séché sous vide, mettre en solution le BnGlu-OCA obtenu à l'exemple 1 (0,81 mmol, 215 mg) dans 5 mL de DCM. Ajouter sous agitation et à température ambiante le n-pentanol (0,08 mmol), 9 µL) puis la DMAP (0,08 mmol, 10 mg). Après 5 min (fin du dégagement de CO₂) la consommation totale du monomère est confirmée par RMN ¹H.

RMN ¹H : 7,30 (m, Ar) ; 5,10 (m, **CH₂**Ar + **CH**OCO) ; 4,30 (dd, J_{HH}=4,2Hz, J_{HH}=7,8Hz, **CH**OH) ; 4,05 (m, O**CH₂**CH₂) ; 2,10-2,50 (m, **CH₂CH₂**CO₂Bn) ; 1,60 (m, OCH₂**CH₂**CH₂) ; 1,30 (m, CH₂CH₂**CH₃**) ; 0,88 (t, JHH=6,8Hz, CH₂CH₂**CH₃**). Incorporation d'un motif n-pentanol pour 9 motifs glutamiques.
GPC : Mn-2060 ; Mw=2560 ; IP= 1,24

### 2) Protection des fonctions alcools terminales

Ajouter au milieu réactionnel 2 équivalents d'anhydride acétique (0,16 mmol, 16 µL) et agiter à température ambiante pendant 1 heure. Le spectre de RMN ¹H confirme la protection totale des fonctions alcools terminales (disparition du signal **CH**OH). Laver le milieu réactionnel par 2 fois 5 mL d'HCl 2N puis 5 mL de saumure. Sécher sur sulfate de sodium anhydre et évaporer à siccité (après co-évaporation au toluène) pour obtenir une huile visqueuse.
RMN ¹H : 7,30 (m, Ar) ; 5,10 (m, **CH₂**Ar + **CH**OCO) ; 4,10 (m, O**CH₂**CH₂) ; 2,05-2,50 (m, **CH₂CH₂**CO₂Bn) ; 2,06 (s, OCO**CH₃**) ; 1,60 (m, OCH₂**CH₂**CH₂) ; 1,30 (m, **CH₂CH₂**CH₃) ; 0,86 (t, J_{HH}=6,8Hz, CH₂CH₂**CH₃**). Disparition du signal **CH**OH et incorporation du motif acétyle, singulet à 2,06 ppm.
GPC : Mn=1960 ; Mw=2430 ; IP= 1,24

### 3) Déprotection des fonctions acides carboxyliques

Hydrogénolyser le copolymère acétylé en solution dans 25 mL d'acétate d'éthyle sous atmosphère d'hydrogène en présence de Pd/C 10% (10 mg) et à pression atmosphérique pendant 1 heure. Filtrer sur célite, rincer à l'acétate d'éthyle et évaporer le solvant. Reprendre au THF et évaporer à siccité. Triturer le polymère dans le chloroforme, éliminer le chloroforme et évaporer à siccité pour obtenir une « mousse » blanche.
RMN ¹H : 5,00-5,30 (m, **CH**OCO) ; 4,02 (m, O**CH₂**CH₂) ; 1,90-2,50 (m, **CH₂CH₂**CO₂H) ; 2,05 (s, OCO**CH₃**); 1,55 (m, OCH₂**CH₂**CH₂) ; 1,22 (m, **CH₂CH₂**CH₃) ; 0,77 (t, J_{HH}=6,8Hz, CH₂CH₂**CH₃**). Débenzylation totale.
GPC : Mn=1860; Mw=2230 ; IP= 1,19

### Copolymérisation statistique lac-OCA / BnGIu-OCA :

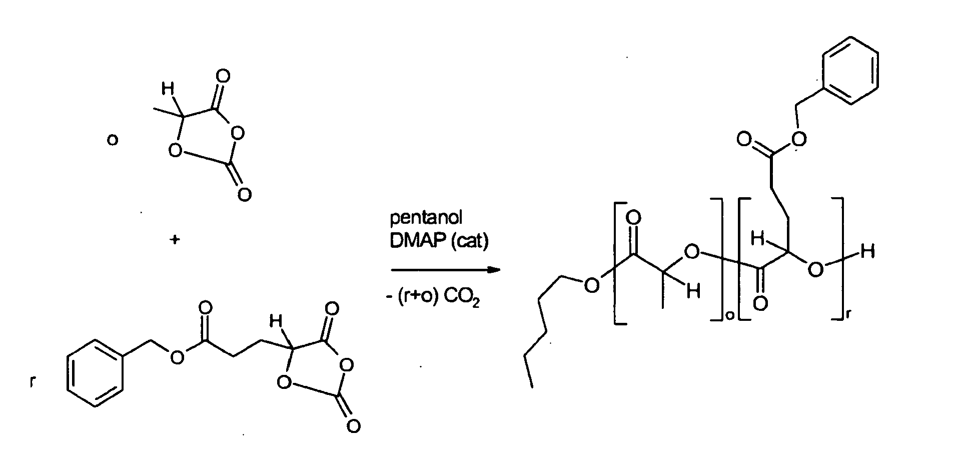

### - poly(Lac)₁₀poly(BnGlu)₁₀

Dans un tube de schlenk préalablement séché sous vide, mettre en solution le BnGlu-OCA obtenu à l'exemple 1 (0,95 mmol, 250 mg) et le Lac-OCA (0,95 mmol, 110 mg) dans 3 mL de DCM. Ajouter sous agitation et à température ambiante le n-pentanol (0,095 mmol, 10 µL) puis la DMAP (0,095 mmol, 12 mg). Après 5 min (fin du dégagement de CO₂) la consommation totale des deux monomères est confirmée par RMN ¹H. Rajouter 5 mL de DCM puis laver le milieu réactionnel par 2 fois 5mL d'HCl 2N et 5 mL de saumure. Sécher sur sulfate de sodium anhydre et évaporer à siccité pour obtenir une huile visqueuse (250 mg, 87%).
RMN ¹H : 7,30 (m, Ar) ; 5,07 (m, **CH₂**Ar + **CH**OCO) ; 4,30 (m, **CH**OH) ; 4,05 (m, OCH₂CH₂); 2,10-2,50 (m, **CH₂CH₂**CO₂Bn); 1,53 (m, **CH₃**CH + OCH₂**CH₂**CH₂) ; 1,23 (m, **CH₂CH₂**CH₃); 0,80 (t, J_{HH}=6,8Hz, CH₂CH₂**CH₃**)**.** Incorporation d'un motif n-pentanol pour 10 motifs glutamiques et 10 motifs lactiques.
GPC : Mn-2800 ; Mw=3860 ; IP=1,38

### - poly(Lac)₁₀poly(BnGlu)₁

Dans un tube de schlenk préalablement séché sous vide, mettre en solution le BnGlu-OCA obtenu dans l'exemple 1 (0,12 mmol, 32 mg) et le Lac-OCA (1,20 mmol, 140 mg) dans 5 mL de DCM. Ajouter sous agitation et à température ambiante le n-pentanol (0,12 mmol, 12 µL) puis la DMAP (0,12 mmol, 14 mg). Après 5 min (fin du dégagement de CO₂) la consommation totale des deux monomères est confirmée par RMN ¹H. Laver le milieu réactionnel par 2 fois 5 mL d'HCl 2N et 5 mL de saumure. Sécher sur sulfate de sodium anhydre et évaporer à siccité pour obtenir une huile visqueuse (90 mg, 81%).
RMN ¹H: 7,30 (m, Ar) ; 5,10 (m, **CH₂**Ar + **CH**OCO) ; 4,30 (m, **CH**OH) ; 4,05 (m, O**CH₂**CH₂); 2,10-2,50 (m, **CH₂CH₂**CO₂Bn); 1,50 (m, **CH₃**CH + OCH₂**CH₂**CH₂) ; 1,20 (m, **CH₂CH₂**CH₃) ; 0,80 (t, J_{HH}=6,8Hz, CH₂CH₂**CH₃**). Incorporation d'un motif pentanol pour 1 motif glutamique et 10 motifs lactiques.
GPC : Mn=1125 ; Mw=1550 ; IP=1,38

### - poly(Lac)₁₀poly(Glu)₂OAc

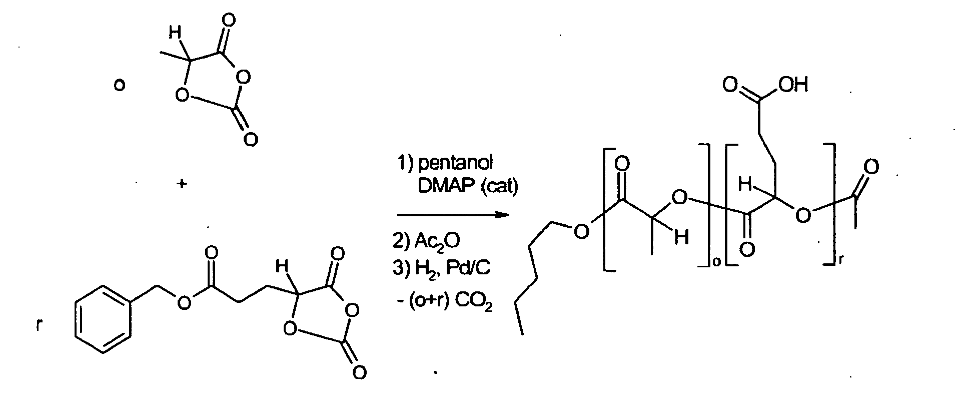

### 1) Polymérisation

Dans un tube de schlenk préalablement séché sous vide, mettre en solution le BnGlu-OCA obtenu à l'exemple 1 (0,45 mmol, 119 mg) et le Lac-OCA (2,33 mmol, 270 mg) dans 10 mL de DCM. Ajouter sous agitation et à température ambiante le n-pentanol (0,23 mmol, 26 µL) puis la DMAP (0,23 mmol, 28 mg). Après 5 min (fin du dégagement de CO₂) la consommation totale des monomères est confirmée par RMN ¹H.
RMN ¹H : 7,30 (m, Ar) ; 5,10 (m, **CH₂**Ar + **CH**OCO) ; 4,30 (m, **CH**OH) ; 4,05 (m, O**CH₂**CH₂); 2,10-2,50 (m, **CH₂CH₂**CO₂Bn) ; 1,50-1,20 (m, **CH₃**CH + OCH₂**CH₂**CH2, **CH₂CH₂**CH₃) 0,80 (t, J_{HH}=6,8Hz, CH₂CH₂**CH₃**). Incorporation d'un motif n-pentanol pour 1,8 motifs glutamiques et 10 motifs lactiques.
GPC : Mn=1560 ; Mw=2070 ; IP=1,33

### 2) Protection des fonctions alcools terminales

Ajouter au milieu réactionnel 2 équivalents d'anhydride acétique (0,46 mmol, 44 µL) et agiter à température ambiante pendant 1 heure. Le spectre de RMN ¹H confirme la protection totale des fonctions alcools terminales (disparition du signal **CH**OH). Laver le milieu réactionnel par 2 fois 10 mL d'HCl 2N puis 5 mL de saumure. Sécher sur sulfate de sodium anhydre et évaporer à siccité (après co-évaporation au toluène) pour obtenir une huile visqueuse (250 mg, 91%).
RMN ¹H : 7,30 (m, Ar) ; 5,10 (m, **CH₂**Ar + C**H**OCO) ; 4,05 (m, O**CH₂**CH₂) ; 2,05-2,50 (m, **CH₂CH**₂CO₂Bn) ; 2,05 (s, OCO**CH₃**) ; 1,50-1,20 (m, **CH₃**CH + OCH₂**CH₂**CH₂, **CH₂CH₂**CH₃) ; 0,80 (t, J_{HH}⁼6,8Hz, CH₂CH₂**CH₃**). Disparition du signal **CH**OH et incorporation d'un motif acétyle, singulet 2,05 ppm.
GPC : Mn=1530 ; Mw=2020 ; IP=1,32

### 3) Déprotection des fonctions acides carboxyliques

Hydrogénolyser le copolymère acétylé (220 mg) en solution dans 25 mL d'acétate d'éthyle sous atmosphère d'hydrogène en présence de Pd/C 10% (10 mg) et à pression atmosphérique pendant 1 heure. Filtrer sur célite, rincer à l'acétate d'éthyle et évaporer le solvant. Reprendre au THF et évaporer à siccité pour obtenir une « mousse » blanche (190 mg).
RMN ¹H : 5,10 (m, **CH**OCO) ; 4,05 (m, O**CH**₂CH₂ ; 2,05-2,50 (m, **CH₂CH₂**CO₂H) ; 2,07 (s, OCO**CH₃**); 1,50-1,20 (m, **CH₃**CH + OCH₂**CH₂**CH₂ + **CH₂CH₂**CH₃) ; 0,80 (t, J_{HH}=6,8Hz, CH₂CH₂**CH₃**). Débenzylation totale.
GPC : Mn=1370 ; Mw=1790 ; IP=1,30

### Copolymérisation par blocs lac-OCA / BnGlu-OCA :

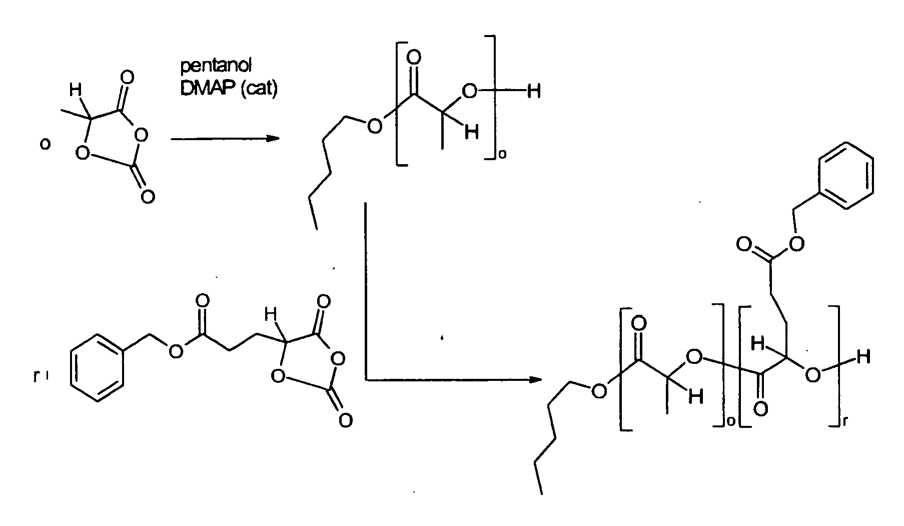

### - poly(Lac)₂₀poly(BnGlu)₁₀

Dans un tube de schlenk préalablement séché sous vide, mettre en solution le Lac-OCA (1,77 mmol, 205 mg) dans 3 mL de DCM. Ajouter sous agitation et à température ambiante le n-pentanol (0,088 mmol, 10 µL) puis la DMAP (0,088 mmol, 10 mg). Après 5 min (fin du dégagement de CO₂) la consommation totale du monomère est confirmée par RMN ¹H.
RMN ¹H : 5,15 (m, **CH**OCO) ; 4,30 (m, **CH**OH) ; 4,10 (m, O**CH₂**CH₂) ; 1,50 (m, **CH₃**CH + OCH₂**CH₂**CH₂) ; 1,30 (m, **CH₂CH₂**CH₃) ; 0,90 (t, J_{HH}=6,8Hz, CH₂CH₂**CH₃**). Incorporation d'un motif n-pentanol pour 20 motifs lactiques.
GPC : Mn=1440 ; Mw=1780 ; IP=1,24

A la solution précédente de poly(lac)₂₀ ajouter une solution de BnGlu-OCA obtenu à l'exemple 1 dans 2 mL de DCM. Après 5 min (fin du dégagement de CO₂) la consommation totale du monomère est à nouveau confirmée par RMN ¹H. La disparition du signal **CH**OH à 4,30 ppm confirme que le poly(lac)₂₀ joue bien son rôle de macro-initiateur. Laver le milieu réactionnel par 2 fois 10 mL d'HCl 2N et 10 mL de saumure. Sécher sur sulfate de sodium anhydre et évaporer à siccité pour obtenir une mousse blanche (330 mg, 88%).
RMN ¹H : 7,30 (m, Ar) ; 5,10 (m, **CH₂**Ar + **CH**OCO) ; 4,30 (m, **CH**OH); 4,10 (m, O**CH₂**CH₂); 2.10-2,50 (m, **CH₂CH₂**CO₂Bn) ; 1,50 (m, **CH₃**CH + OCH₂**CH₂**CH₂) ; 1,30 (m, **CH₂CH₂**CH₃); 0,90 (t, J_{HH}=6,8Hz, CH₂CH₂**CH₃**). Incorporation de l'alcool terminal du poly(Lac)₂₀ dans la chaîne du poly(BnGlu). Présence d'un motif n-pentanol pour 10 motifs glutamiques et 20 motifs lactiques.
GPC : Mn=2540 ; Mw=3120 ; IP=1,23

### Exemple 3 : Synthèse de l'OCA- BnSerine

### Diazotation de la L-Bn-serine (Deechongkit, S. et al., Org. Lett 2004, 6, 497-500)

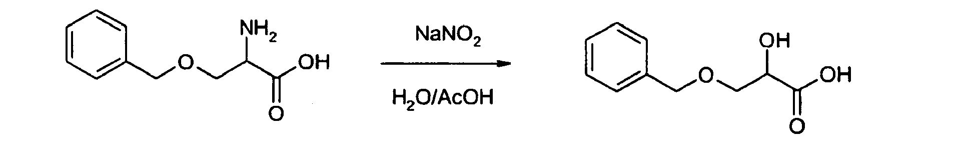

Ajouter en 30 min et à 0°C 20 mL de solution aqueuse de NaNO₂ 2M (40,0 mmol) à une suspension de L-BnSerine (3,92 g ; 20,0 mmol) dans 200 mL d'un mélange H₂O/AcOH 8/2. le milieu réactionnel est homogène après 1 h à 0°C. Agiter 5 h à température ambiante pour terminer la réaction (contrôle CCM : DCM 90 - MeOH 9 - AcOH 1). Ajouter 250 mL d'eau et extraire à l'AcOEt (4 x 100 mL). Rassembler les phases organiques et les laver à la saumure puis sécher sur sulfate de sodium. Evaporer le solvant pour obtenir 3,85 g d'huile. Purifier par chromatographie (DCM 95 - MeOH 4,5 - AcOH 0,5). Récupérer une huile qui cristallise (3,15 g ; 82%).
RMN¹ H (CDCl₃ - 300 MHz) : 3,80 (m, 2H) ; 4,41 (m, 1H) ; 4,57 (d, 1H, J_{HH} = 12,3 Hz) ; 4,62 (d, 1H, J_{HH} = 12,3 Hz) ; 7,33 (m, 5H)
RMN ¹³C. (CDCl₃ - 75 MHz) : 70,4 ; 71,0 ; 73,6 ; 127,9-128,5 ; 137,2 ; 176,2.
Point de fusion : 55-56°C

### Synthèse de l'OCA BnSerine :

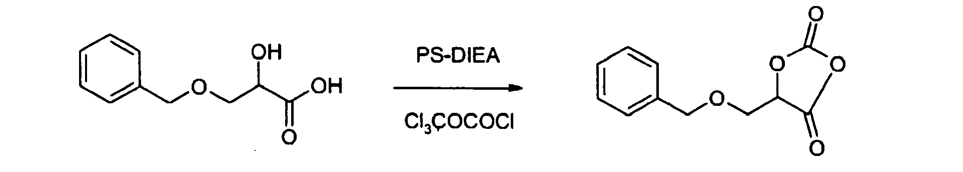

Ajouter à une solution d'hydroxyacide (240 mg ; 1,22 mmol) dans 5 mL de THF anhydre la diisopropyléthylamine supportée (400 mg ; 1,22 mmol). Ajouter à 0°C le diphosgène (1,22 mmol ; 0,15 mL) et agiter le milieu réactionnel à température ambiante pendant 4 heures. Filtrer et rincer la résine puis évaporer le THF. Laver le résidu par du pentane (2 x 5 mL) et évaporer à siccité pour obtenir une huile incolore (240 mg ; 88%).
RMN ¹H (CDCl₃ - 300 MHz) : 3,84 (m, 2H) ; 4,49 (d, 1H, J_{HH} = 12,1 Hz) ; 4,54 (d, 1H, J_{HH} = 12,1 Hz) ; 5,03 (m, 1H) ; 7,17-7,31 (m, 5H).
RMN ¹³C (CDCl₃ - 75 MHz) : 66,1 ; 73,8 ; 79,6 ; 127,7 ; 128,3 ; 128,7 ; 136,2 ; 148,4; 165,5.
Point de fusion : 63-64°C.
IR (cm⁻¹ ;CCl₄): 1905, 1815.
MS (DCI, CH₄) : 222 ; 198 ; 181 ; 131 ; 119; 107. (M=222,19)

### Exemple 4 :Synthèse de l'OCA-CbzLysine

### Diazotation de la L-Cbzlysine (Deechongkit, S. et al., Org. Lett. 2004, 6, 497-500; Shin, I, et al. J. Org. Chem. 2000, 65, 7667-7675).

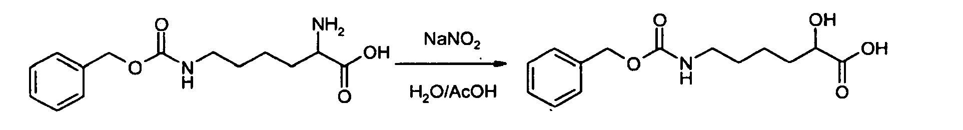

Ajouter en 30 min 400 mL de solution de NaNO₂ 2M (5,0 g ; 70,0 mmol) à une suspension de L-CBzLysine (10,0 g ; 35,7 mmol, Bachem) dans 200 mL d'un mélange H₂O/AcOH 1/1 refroidi sur bain de glace. Le milieu réactionnel est agité à température ambiante pendant 18 heures et est alors homogène.

Ajouter 200 mL d'eau et extraire à l'acétate d'éthyle (3 x 150 mL). Rassembler les phases organiques et les laver à la saumure (3 x 100 mL). Sécher sur sulfate de sodium. Evaporer les solvants puis reprendre le résidu huileux au toluène pour éliminer le maximum d'acide acétique. Evaporer à siccité pour obtenir 11,5 g d'huile jaune (majoritairement le dérivé O-acétylé).

Réaliser la déacétylation dans 100 mL d'un mélange MeOH-H₂O 1/1 en présence de 6,0 g de carbonate de potassium (pH 9-10). Réaction terminée après 3h30. Ajouter 50 mL d'eau et évaporer le méthanol. Extraire la phase aqueuse par 100 mL d'acétate d'éthyle. Acidifier la phase aqueuse (pH 2) et extraire à l'acétate d'éthyle (2 x 100 mL). Laver à la saumure et sécher sur sulfate de sodium. Evaporer pour obtenir 8,70 g de poudre blanchâtre collante. Triturer dans 100 ml de pentane. Eliminer le solvant pour obtenir une poudre blanche (7,22 g ; 72%).
RMN ¹H (CDCl₃ - 300 MHz) : 1,51 (m, 4H), 1,72-1,76 (m, 2H), 3,18 (m, 2H), 4,25 (m, 1 H), 5,11 (s, 2H), 7,34 (m, 5H).
RMN ¹³C (CDCl3 - 75 MHz) : 21,9 ; 29,3 ; 33,5 ; 40,9 ; 66,9 ; 70,1 ; 128,0-128,5 ; 136,4 ; 158,0 ; 177,7.
Point de fusion : 74-76°C (lit 79-81°C).

### Synthèse de l'OCA-CbzLysine :

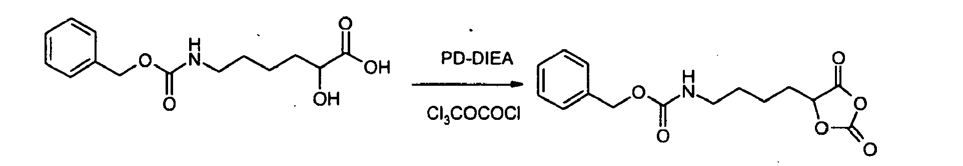

Ajouter à une solution d'hydroxyacide (160 mg ; 0,57 mmol) dans 3 mL de THF anhydre la diisopropyléthylamine supportée (380 mg ; 1,14 mmol). Ajouter à 0°C le diphosgène (0,57 mmol ; 70 µL) et agiter le milieu réactionnel à température ambiante pendant 1 heures. Filtrer et rincer la résine puis évaporer le THF. Laver le résidu au pentane (2 x 5 mL) et évaporer à siccité pour obtenir une huile incolore (160 mg; 91%).
RMN ¹H (CDCl₃ - 300 MHz) : 1,55 (m, 4H) ; 2,10 (m, 2H) ; 3,22 (m, 2H) ; 4,80 (m, 1H) ; 4,95 (m,1H), 5,10 (s, 2H) ; 7,35 (m, 5H).
RMN ₁₃C (CDCl3 - 75 MHz) : 21,3 ; 29,1 ; 30,3 ; 40,3 ; 66,7 ; 79,6 ; 128,1 ; 128,2 ; 128,6 ; 136,6 ; 148,3 ; 156,6; 167,1.
Point de fusion : 57-58°C.
JR (cm⁻¹; CCl₄) : 1896, 1812, 1718.
MS (DCI, CH₄) : 354 ; 325 ; 308 ; 290 ; 264 ; 218 ; 192 ; 174 ; 156 ; 119. (M = 307,29)

## Revendications

1. OCA de formule générale I suivante : 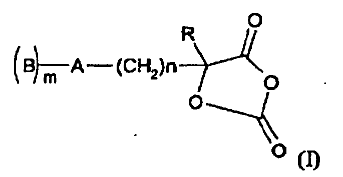 dans laquelle :
- n est un nombre entier compris entre 1 et 10, avantageusement entre 1 et 6 ;
- A représente un hétéroatome choisi parmi O, N et S ou un radical -COO, ou un radical NH, en particulier A représente NH, O ou un radical -COO ;
- B représente un groupe protecteur de A, en particulier B représente un groupe benzyloxycarbonyle lorsque A représente NH ou B représente un groupe benzoyle lorsque A représente O ou un groupe -COO ;
- m vaut 1 ou 2
- R représente un radical choisi dans le groupe constitué par
. l'hydrogène,
. un radical (CH₂)n-A-(B)m dans lequel n, m, A et B sont tels que définis précédemment,
. les radicaux alkyles en C₁-C₁₂, linéaire ou ramifiés, saturés ou insaturés,
. les radicaux aralkyles en C₇-C₂₀,
. les radicaux cycloalkyles, simples ou fusionnés, en C₃-C₁₄,
. les radicaux hétérocycloalkyles, simple ou fusionnés en C₂-C₁₄,
. les radicaux aromatiques, simples ou fusionnés, en C₆-C₁₄, et
. les radicaux hétéroaromatiques, simples ou fusionnés en C₃-C₁₄;
en particulier R représente H,
ou leurs sels d'addition, leurs isomères, énantiomères, diastéréoisomères ou leurs mélanges.

2. OCA selon la revendication 1, **caractérisé en ce qu'**il est choisi parmi :

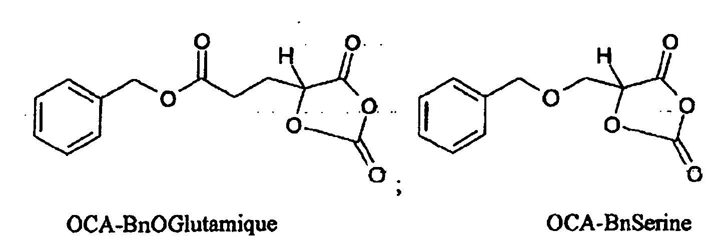

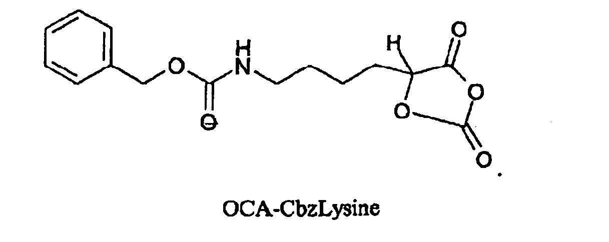

3. Utilisation d'un OCA de formule générale (I) selon l'une quelconque des revendications 1 et 2 pour la préparation d'un poly(α-hydroxyacides).

4. Procédé de préparation de poly(α-hydroxyacides) comprenant les étapes successives suivantes :
i) polymérisation de manière contrôlée de monomères d'OCAs de formule (I) selon l'une quelconque des revendications 1 à 3 dans un solvant organique, avantageusement anhydre, à une température comprise entre -20 et 200°C, avantageusement entre 0 et 100°C, encore plus avantageusement entre 20 et 50°C, en présence d'un système catalytique comprenant une base, ladite base étant un hétérocycle aromatique à 5 ou 6 chaînons comprenant au moins un atome d'azote endocyclique, sous réserve que lorsque la base est utilisée seule dans le système catalytique, elle ne représente pas la pyridine, la 2-méthylpyridine, la 2,6-diméthylpyridine ou la 2-méthoxypyridine; puis
ii) le cas échéant purification du polymère obtenu à l'étape i),
iii) récupération du polymère obtenu à l'étape i) ou ii),
iv) éventuellement protection du groupe OH terminal du polymère obtenu à l'étape iii) avec un groupe O-protecteur différent de B ;
v) éventuellement déprotection du groupe A du polymère obtenu à l'étape iv) afin d'obtenir le groupe AH ;
vi) éventuellement déprotection du groupe OH terminal du polymère obtenu à l'étape v) ;

5. Procédé selon la revendication 4, **caractérisé en ce que** ladite base est une amino-pyridine de formule (II) 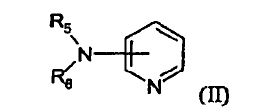 dans laquelle R₅ et R₆ représentent indépendamment l'un de l'autre un radical alkyle en C₁-C₆, linéaire ou ramifié, saturé ou insaturé, ou R₅ et R₆ forment ensemble un hétérocycloalkyle à 5 ou 6 chaînons, le groupe -NR₅R₆ étant en position 2 ou 4, en particulier ladite base est la 4-diméthylamino pyridine.

6. Procédé selon l'une quelconque des revendications 4 et 5, **caractérisé en ce que** le système catalytique de l'étape i) comprend en outre un réactif protique, ledit réactif protique étant avantageusement choisi dans le groupe constitué par l'eau, les alcools, les amines primaires et secondaires, les thiols et les polymères à fonctionnalité alcool, amino et thiol.

7. Procédé selon la revendication 6, **caractérisé en ce que** le réactif protique est choisi dans le groupe constitué par l'eau, les alcools aliphatiques en C₁-C₁₂, les poly(acide glycolique), les poly(acide lactique) et leurs copolymères.

8. Procédé selon l'une quelconque des revendications 4 à 7, **caractérisé en ce que**, lors de l'étape i), le solvant organique est choisi dans le groupe constitué par les solvants aliphatiques chlorés, les éthers, les éthers cycliques, les aromatiques, les amides ou les lactames.

9. Poly(α-hydroxyacide) de formule (IV) 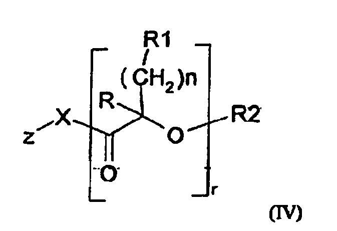 dans laquelle
• n et R sont tels que définis dans l'une quelconque des revendications 1 et 2
• R1 représente le groupe -A-(B)m ou le groupe -A-H, dans lesquels A, B et m sont tels que définis dans l'une quelconque des revendications 1 à-5 ;
• R2 représente un groupe O-protecteur différent de B ou un atome d'hydrogène;
• X représente :
- un hétéroatome, choisi dans le groupe constitué par O, N et S, avantageusement O, ou
- un polymère choisi dans le groupe constitué par les poly(acide glycolique), les poly(acide lactique) et leurs copolymères, ledit polymère étant terminé par un radical -X' dans lequel X' représente un hétéroatome, choisi dans le .groupe constitué par O, N et S, avantageusement O
ou répondant à la formule suivante : 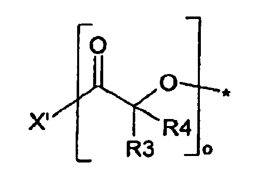 dans laquelle o est supérieur ou égal à 1, R3 et R4 représentent H ou un radical alkyle en C₁-C₆, avantageusement CH₃, cycloalkyle en C₃-C₆ ou aryl(alkyle en C₁-C₆), X' représente un hétéroatome choisi dans le groupe constitué par O, N et S, avantageusement O, X' étant lié au radical Z dans la formule (IV) ;
• Z représente un radical alkyle en C₁-C₁₂, linéaire ou ramifié, saturé ou insaturé
• r est supérieur ou égal à 1.

10. Poly(α-hydroxyacide) selon la revendication 9, **caractérisé en ce que** r est compris entre 1 et 500, avantageusement entre 1 et 400, encore plus avantageusement entre 1 et 50.

11. Poly(a-hydroxyacide) selon la revendication 9 ou 10, **caractérisé en ce que** X représente l'hétéroatome O et Z représente un radical alkyle en C₁-C₆.

12. Poly(α-hydroxyacide) selon la revendication 9, **caractérisé en ce que** X' représente l'hétéroatome O et Z représente un radical alkyle en C₁-C₆.

13. Poly(α-hydroxyacide) selon l'une quelconque des revendications 9 à 12, **caractérisé en ce que** ledit poly(α-hydroxyacide) a un indice de polydispersité compris entre 1 et 1,5

14. Utilisation d'un poly(α-hydroxyacide) selon l'une quelconque des revendications 9 à 13, pour la vectorisation de principes actifs.

15. Utilisation d'un poly(α-hydroxyacide) selon l'une quelconque des revendications 9 à 13, pour la fabrication de biomatériaux.

## Claims

1. OCA of following general formula I: 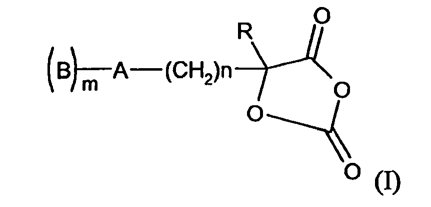 in which:
- n is an integer between 1 and 10, advantageously between 1 and 6;
- A is a heteroatom chosen from among O, N and S, or a -COO radical or -NH radical, in particular A is NH, O or a -COO radical;
- B is a protecting group of A, in particular B is a benzyloxycarbonyl group when A is NH, or B is a benzyl group when A is O or a -COO group;
- m has a value of 1 or 2,
- R is a radical chosen from among the group consisting of:
. hydrogen,
. a (CH₂) n-A- (B)m radical in which n, m, A and B are such as previously defined,
. straight or branched, saturated or unsaturated, C₁ to C₁₂ alkyl radicals,
. C₇ to C₂₀ aralkyl radicals,
. simple or fused, C₃ to C₁₄ cycloalkyl radicals,
. simple or fused, C₂ to C₁₄ heterocycloalkyl radicals,
. simple or fused, C₆ to C₁₄ aromatic radicals, and
. simple or fused, C₃ to C₁₄ heteroaromatic radicals;
in particular R represents H,
or their addition salts, their isomers, enantiomers, diastereoisomers or mixtures thereof.

2. OCA according to claim 1, **characterized in that** it is chosen from among: 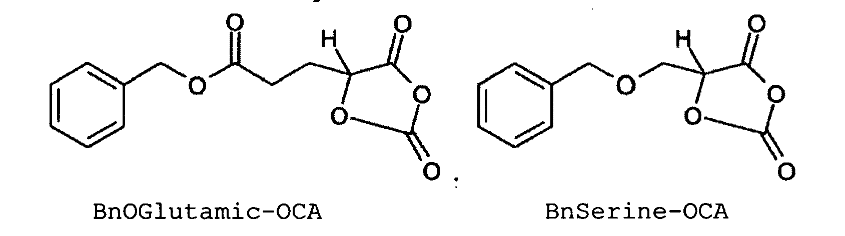 and

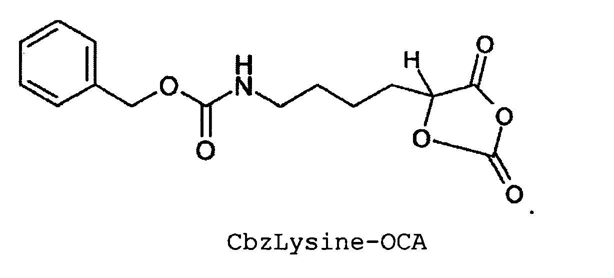

3. Use of an OCA of general formula (I) according to either of claims 1 and 2 for the preparation of a poly(α-hydroxy acid).

4. Method to prepare poly(α-hydroxy acids) comprising the following successive steps:
i) controlled polymerization of monomers of formula (I) OCAs according to any of claims 1 to 3 in an organic, advantageously anhydrous, solvent at a temperature of between -20 and 200°C, advantageously between 0 and 100°C, more advantageously between 20 and 50°C, in the presence of a catalytic system comprising a base, said base being a 5- or 6-membered aromatic heterocycle containing at least one endocyclic nitrogen atom, provided that when the base is used alone in the catalytic system it does not represent pyridine, 2-methylpyridine, 2,6-dimethylpyridine or 2-methoxypyridine, then
ii) if need be, purification of the polymer obtained at step i),
iii)collection.of the polymer obtained at step i) or ii),
iv) optional protection of the terminal OH group of the polymer obtained at step iii) by an O-protecting group different from B,
v) optional deprotection of the A group of the polymer obtained at step iv) to obtain the AH group,
vi) optional deprotection of the terminal OH group of the polymer obtained at step v).

5. The method according to claim 4, **characterized in that** said base is an amino-pyridine of formula (II): 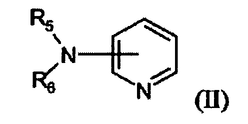 in which R₅ and R₆ independently of each other represent a straight or branched, saturated or unsaturated, C₁-C₆ alkyl radical, or R₅ and R₆ together form a 5 or 6 membered heterocycloalkyl, the -NR₅R₆ group being at position 2 or 4, in particular said base is 4-dimethylamino pyridine.

6. The method according to either of claims 4 and 5, **characterized in that** the catalytic system of step i) also comprises a protuc reagent, said protic reagent advantageously being chosen from the group consisting of water, alcohols, primary and secondary amines, thiols and polymers with alcohol, amino and thiol function.

7. The method according to claim 6, **characterized in that** the protic reagent is chosen from the group consisting of water, C₁-C₁₂ aliphatic alcohols, polyglycolic acid, poly-lactic acid and their copolymers.

8. The method according to any of claims 4 to 7, **characterized in that** during step i) the organic solvent is chosen from the group consisting of chlorinated aliphatic solvents, ethers, cyclic ethers, aromatics, amides or lactames.

9. Poly (α-hydroxy acid) of formula (IV): 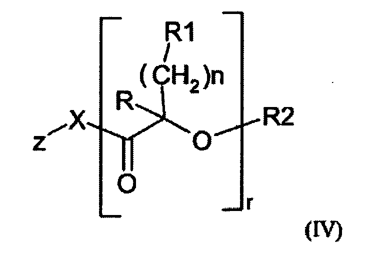 in which:
• n and R are such as defined in either of claims 1 and 2,
• R1 represents the -A-(B)m group or -A-H group, in which A, B and m are such as defined in any of claims 1 to 5,
• R2 represents an O-protecting group different from B, or a hydrogen atom;
• X represents:
- a heteroatom, chosen from the group consisting of O, N and S, advantageously O, or
- a polymer chosen from the group consisting of polyglycolic acid, polylactic acid and their copolymers, said polymer being terminated by an -X' radical in which X' is a heteroatom chosen from the group consisting of O, N and S, advantageously O;
or meeting the following formula: 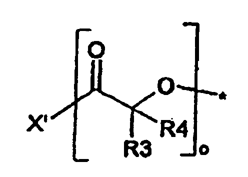 in which O is equal to or greater than 1, R3 and R4 represent H or C₁-C₆ alkyl radical, advantageously CH₃, C₃-C₆ cycloalkyl or aryl (C₁-C₆)alkyl, X' represents a heteroatom chosen from the group consisting of O, N and S, advantageously O, X' being bound to the radical Z in formula (IV);
• Z represents a straight or branched, saturated or unsaturated, C₁-C₁₂ alkyl radical ;
• r is equal to or greater than 1,

10. Poly(α-hydroxy acid) according to claim 9, **characterized in that** r lies between 1 and 500, advantageously between 1 and 400, further advantageously between 1 and 50.

11. Poly(α-hydroxy acid) according to claim 9 or 10, **characterized in that** X represents the heteroatom O, and Z represents a C₁-C₆ alkyl radical.

12. Poly(α-hydroxy acid) according to claim 9, **characterized in that** X' represents the heteroatom O and Z represents a C₁-C₆ alkyl radical.

13. Poly(α-hydroxy acid) according to any of claims 9 to 12, **characterized in that** said poly(α-hydroxy acid) has a polydispersity index of between 1 and 1.5.

14. Use of a poly(α-hydroxy acid) according to any of claims 9 to 13, for the vectoring of active ingredients.

15. Use of a poly(α-hydroxy acid) according to any of claims 9 to 13, for the manufacture of biomaterials.

## Patentansprüche

1. OCA der folgenden allgemeinen Formel I: 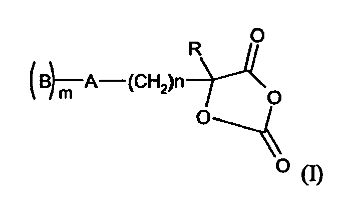 wobei:
- n eine ganze Zahl zwischen 1 und 10, vorzugsweise zwischen 1 und 6 ist,
- A ein Heteroatom, ausgewählt aus O, N und S, oder einen Rest -COO oder einen Rest NH bedeutet, wobei A insbesondere NH, O oder einen Rest -COO bedeutet;
- B eine Schutzgruppe für A bedeutet, wobei B insbesondere einen Benzyloxycarbonylrest bedeutet, wenn A für NH steht, oder B einen Benzylrest bedeutet, wenn A für O oder einen Rest -COO steht;
- m gleich 1 oder 2 ist;
- R einen Rest, ausgewählt aus der Gruppe bestehend aus
- Wasserstoff,
- einem Rest (CH₂)n-A-(B)m, wobei n, m, A und B wie vorstehend definiert sind,
- geradkettigen oder verzweigten, gesättigten oder ungesättigten C₁-C₁₂-Alkylresten,
- C₇-C₂₀-Aralkylresten,
- einzelnen oder kondensierten C₃-C₁₄-Cycloalkylresten,
- einzelnen oder kondensierten C₂-C₁₄-Heterocycloalkylresten,
- einzelnen oder kondensierten aromatischen C₆-C₁₄-Resten und
- einzelnen oder kondensierten heteroaromatischen C₃-C₁₄-Resten; bedeutet,
R insbesondere H bedeutet,
oder ihre Additionssalze, ihre Isomere, Enantiomere, Diastereoisomere oder ihre Gemische.

2. OCA nach Anspruch 1, **dadurch gekennzeichnet, dass** es ausgewählt ist aus: 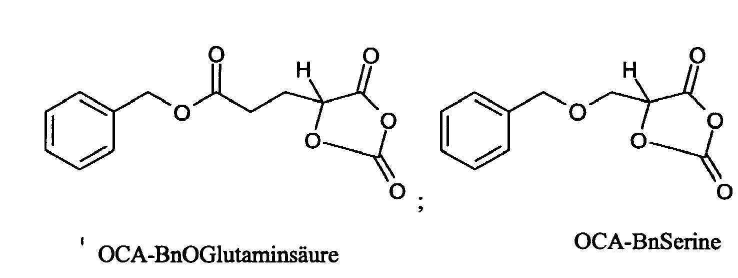 et
und

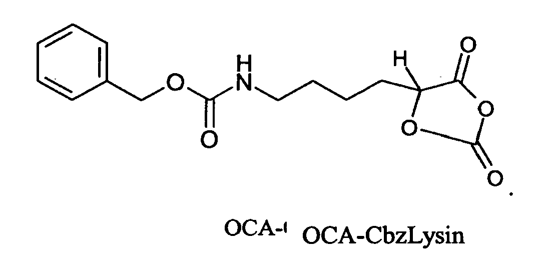

3. Verwendung eines OCA der allgemeinen Formel (I) nach einem der Ansprüche 1 und 2 zur Herstellung einer Poly(α-hydroxysäure).

4. Verfahren zur Herstellung von Poly(α-hydroxysäuren), umfassend die folgenden aufeinanderfolgenden Schritte:
i) kontrollierte Polymerisation von OCA-Monomeren der Formel (I) nach einem der Ansprüche 1 bis 3 in einem organischen, vorzugsweise wasserfreien Lösungsmittel bei einer Temperatur zwischen -20 und 200°C, vorzugsweise zwischen 0 und 100°C, noch mehr bevorzugt zwischen 20 und 50°C, in Gegenwart eines katalytischen Systems, das eine Base umfasst, wobei die Base ein 5- oder 6-gliedriger aromatischer Heterocyclus ist, der mindestens ein endocyclisches Stickstoffatom umfasst, mit der Maßgabe, dass wenn die Base allein in dem katalytischen System verwendet wird, sie nicht Pyridin, 2-Methylpyridin, 2,6-Dimethylpyridin oder 2-Methoxypyridin bedeutet; dann
ii) gegebenenfalls Reinigung des in Schritt i) erhaltenen Polymers,
iii) Gewinnung des in Schritt i) oder ii) erhaltenen Polymers,
iv) gegebenenfalls Schützen der endständigen OH-Gruppe des in Schritt iii) erhaltenen Polymers mit einer von B verschiedenen O-Schutzgruppe;
v) gegebenenfalls Entfernen der Schutzgruppe von dem Rest A des in Schritt iv) erhaltenen Polymers, um den Rest AH zu erhalten;
vi) gegebenenfalls Entfernen der Schutzgruppe von der endständigen OH-Gruppe des in Schritt v) erhaltenen Polymers.

5. Verfahren nach Anspruch 4, **dadurch gekennzeichnet, dass** die Base ein Aminopyridin der Formel (II) ist 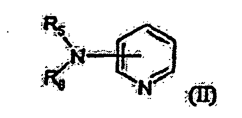 wobei R₅ und R₆ unabhängig voneinander einen geradkettigen oder verzweigten, gesättigten oder ungesättigten C₁-C₆-Alkylrest bedeuten oder R₅ und R₆ zusammen einen 5- oder 6-gliedrigen Heterocycloalkylrest bilden, wobei der Rest -NR₅R₆ in Position 2 oder 4 vorliegt, wobei die Base
insbesondere 4-Dimethylaminopyridin ist.

6. Verfahren nach einem der Ansprüche 4 und 5, **dadurch gekennzeichnet, dass** das katalytische System von Schritt i) ferner ein protisches Reagens umfasst, wobei das protische Reagens vorzugsweise aus der Gruppe bestehend aus Wasser, Alkoholen, primären und sekundären Aminen, Thiolen und Polymeren mit Alkohol-, Amino- und Thiolfunktion ausgewählt ist.

7. Verfahren nach Anspruch 6, **dadurch gekennzeichnet, dass** das protische Reagens aus der Gruppe bestehend aus Wasser, aliphatischen C₁-C₁₂-Alkoholen, Poly(glycolsäure), Poly(milchsäure) und ihren Copolymeren ausgewählt ist.

8. Verfahren nach einem der Ansprüche 4 bis 7, **dadurch gekennzeichnet, dass** in Schritt i) das organische Lösungsmittel aus der Gruppe bestehend aus chlorierten aliphatischen Lösungsmitteln, Ethern, cyclischen Ethern, aromatischen Substanzen, Amiden oder Lactamen ausgewählt ist.

9. Poly(α-hydroxysäure) der Formel (IV) 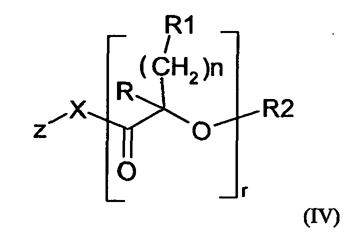 wobei
• n und R wie in einem der Ansprüche 1 und 2 definiert sind;
• R1 den Rest A-(B)m oder den Rest -A-H bedeutet, wobei A, B und m wie in einem der Ansprüche 1 bis 5 definiert sind;
• R2 eine von B verschiedene O-Schutzgruppe oder ein Wasserstoffatom bedeutet;
• X:
- ein Heteroatom, ausgewählt aus der Gruppe bestehend aus O, N und S, vorzugsweise O, oder
- ein Polymer, ausgewählt aus der Gruppe bestehend aus Poly(glycolsäure), Poly(milchsäure) und ihren Copolymeren, wobei das Polymer einen endständigen Rest -X' aufweist, wobei X' ein Heteroatom, ausgewählt aus der Gruppe bestehend aus O, N und S, vorzugsweise O, bedeutet, oder die folgende Formel aufweist: 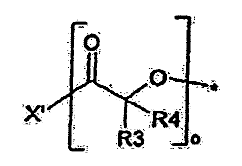 wobei o größer als oder gleich 1 ist, R3 und R4 H oder einen C₁-C₆-Alkylrest, vorzugsweise CH₃, C₃-C₆-Cycloalkyl oder Aryl-C₁-C₆-alkyl bedeuten; X' ein Heteroatom, ausgewählt aus der Gruppe bestehend aus O, N und S, vorzugsweise O, bedeutet, wobei X' an Rest Z in Formel (IV) gebunden ist;
bedeutet;
• Z einen geradkettigen oder verzweigten, gesättigten oder ungesättigten C₁-C₁₂-Alkylrest bedeutet;
• r größer als oder gleich 1 ist.

10. Poly(α-hydroxysäure) nach Anspruch 9, **dadurch gekennzeichnet, dass** r zwischen 1 und 500, vorzugsweise zwischen 1 und 400, noch mehr bevorzugt zwischen 1 und 50 beträgt.

11. Poly(α-hydroxysäure) nach Anspruch 9 oder 10, **dadurch gekennzeichnet, dass** X das Heteroatom O darstellt und Z einen C₁-C₆-Alkylrest bedeutet.

12. Poly(α-hydroxysäure) nach Anspruch 9, **dadurch gekennzeichnet, dass** X' das Heteroatom O darstellt und Z einen C₁-C₆-Alkylrest bedeutet.

13. Poly(α-hydroxysäure) nach einem der Ansprüche 9 bis 12, **dadurch gekennzeichnet, dass** die Poly(α-hydroxysäure) einen Polydispersitätsindex zwischen 1 und 1,5 aufweist.

14. Verwendung einer Poly(α-hydroxysäure) nach einem der Ansprüche 9 bis 13 zur Vektorisierung von Wirkstoffen.

15. Verwendung einer Poly(α-hydroxysäure) nach einem der Ansprüche 9 bis 13 zur Herstellung von Biomaterialien.
